# EUROPEAN PATENT SPECIFICATION

(11) **EP 3 573 678 B1**
(45) Date of publication and mention of the grant of the patent: **06.01.2021**
(21) Application number: 18701485.7
(22) Date of filing: 25.01.2018
(51) Int. Cl.: A61M 1/14

(54) **MEDICAL FLUID-CONDUCTING CASSETTE WITH CONTACT PROTECTION**
MEDIZINISCHE FLUIDLEITENDE KASSETTE MIT KONTAKTSCHUTZ
CASSETTE DE CONDUITE DE FLUIDES MÉDICALES AVEC PROTECTION DE CONTACT

(30) Priority: 30.01.2017 EP 17153740
(43) Date of publication of application: 04.12.2019
(73) Proprietor: Fresenius Medical Care Deutschland GmbH, 61352 Bad Homburg (DE)
(72) Inventor: FINI, Massimo, 41037 Mirandola (Modena) (IT); REITER, Reinhold, 26013 Crema (IT); PATRINI, Edoardo, 26010 CasalettoVaprio (IT)
(74) Representative: Bobbert & Partner Patentanwälte PartmbB
(86) International application number: PCT/EP2018/051902
(87) International publication number: WO 2018/138233

(56) References cited:
- EP-A1- 2 745 859
- DE-A1-102007 042 964
- DE-A1-102014 102 598
- US-A1- 2011 064 608

## Description

The present invention relates to a medical fluid-conducting cassette for a medical fluid treatment according to claim 1, for example a blood cassette such as can be used in dialysis devices. It also relates to a method for producing a medical fluid-conducting cassette for a medical fluid treatment or for covering a hard body thereof according to claim 12.

The handling of fluid-conducting cassettes, for example blood cassettes, by medical personnel always bears the risk that, during unpacking of a cassette from the sterile packaging, connection points which are used for adding a fluid into the cassette during the treatment of the patient (in short: fluid addition point of the cassette) are accidentally touched by a finger or another object and are thus contaminated. From EP 2 736 562 B1 a contact protection apparatus is known that can be used to cover the connection point until it is used. DE102007042964 discloses a cassette, wherein fluid channels are covered by a film having an internal structure.

One object of the present invention is to propose a further medical fluid-conducting cassette for a medical fluid treatment comprising both a connection point and a covering device for covering that connection point of the medical fluid-conducting cassette.

The object according to the invention is accomplished by a cassette with the features of claim 1. It is further accomplished by a method with the features of claim 12.

The cassette according to the invention, in particular for conducting a medical fluid, in particular a medical fluid-conducting cassette (short: cassette hereinafter), comprises a hard part, main body or hard body (hereinafter short: hard body), e. g. made from hard resin.

The hard body comprises channels, or parts of channels, for guiding fluids through, or within, the cassette.

The hard body further comprises a first film section (or flexible sheet). The first film section is arranged to cover parts of the hard body. At the same time, it is arranged to cover or form at least one of the channels and/or an interior of the cassette.

The hard body further comprises at least one connection point. The connection point is configured to connect for example tubing in order to provide a fluid communication between at least the channel and an exterior of the cassette.

The hard body further comprises at least one covering device. The covering device is attached to the hard body and arranged so as to cover the connection point. The covering device is attached to the hard body in a releasable or removable manner such that it can be removed from the cassette by a user when using the cassette.

Also, the covering device is a second film section (or flexible sheet) made from the same film material as the first film section. Alternatively, the covering device comprises such a second film section.

A method according to the present invention relates to covering a hard body of a cassette or medical fluid-conducting cassette for a medical fluid treatment with a film material.

The method comprises providing a cassette, the cassette comprising: a hard body comprising channels for guiding fluids through, or within, the cassette; at least one connection point configured to connect at least one of the channels with an exterior of the cassette in a fluid communication.

The method comprises also the steps: attaching a first film section to the hard body so as to cover parts of the hard body and so as to cover at least one of the channels against an exterior of the cassette; attaching a second film section to the hard body so as to cover the connection point in a releasable or removable manner, wherein the second film section comprises the same material as the first film section or consists thereof.

Advantageous developments of the present invention are each also subject-matter of the dependent claims.

In the following and in all of the following embodiments, the use of the expression "may be" or "may have" and so on, is to be understood synonymously with "preferably is" or "preferably has", respectively, and so on, and is intended to illustrate exemplary embodiments according to the invention.

Whenever numerical words are mentioned herein, the person skilled in the art shall recognize or understand them as indications of numerical lower limits. Unless it leads the person skilled in the art to an evident contradiction, the person skilled in the art shall comprehend the specification for example of "one" encompassing "at least one". This understanding is also equally encompassed by the present invention as the interpretation that a numeric word, for example, "one" may alternatively mean "exactly one", wherever this is evidently technically possible for the person skilled in the art. Both are encompassed by the present invention and apply herein to all used numerical words.

Embodiments according to the invention may comprise some or all of the following features.

According to the present invention the covering device is releasable or removable from the hard body without any tools or without being destructive beyond a pre-determined degree or measure.

In some exemplary embodiments according to the present invention the first film section and the second film section are integrally formed or produced, e. g. as a common piece of film material.

In some exemplary embodiments according to the present invention the first film section and the second film section are linked one to the other by at least one perforation section, e.g. a perforation line. Alternatively, or in addition, the first film section and the second film section are linked one to the other by at least two connectors which are integral with both the first film section and the second film section. The connectors preferably being spaced from each other, e. g. by spaces or gaps between corresponding sections thereof.

In some exemplary embodiments according to the present invention the first film section and the second film section are being welded or have been welded one to the other.

In some exemplary embodiments according to the present invention the first film section and the second film section are being glued or have been glued one to the other.

In some exemplary embodiments according to the present invention both the first film section and the second film section are welded or are being welded to the hard body.

In some exemplary embodiments hereof, the welding method or intensity used (or being used) for welding the two film sections to the hard body differ for the first and the second film section. For example, the second film section may be welded or may have been welded to the hard body while using less energy for the welding process. It may be welded (or may have been welded) by a thinner welding seam or line. It may be welded or may have been welded not along a line but by some welding spots or points only. It may be welded or may have been welded by not forming a tight seal between the covered area of the hard body and the exterior.

In some exemplary embodiments according to the present invention the second film section comprises an interconnecting element configured for connecting the second film section to a transport packaging of the cassette.

The transport packaging may be the packaging of the cassette that ensures that the cassette remains sterile until the packaging is opened and the cassette thus is exposed to the unsterile environment or exterior.

In some exemplary embodiments according to the present invention the interconnecting element is being attached (or may have been attached) to the second film section only, that is, it is not being attached (or has not being attached) to the first film section at the same time.

In some exemplary embodiments according to the present invention the interconnecting element may be attached to the side of the second film section facing the exterior of the cassette and/or to the side of the second film section not facing the hard body side.

In some exemplary embodiments according to the present invention the cassette is packed into a transport packaging. The second film section is connected to the transport packaging of the cassette by the interconnecting element.

In some exemplary embodiments according to the present invention the interconnecting element is an adhesive strip, material or layer.

In some exemplary embodiments according to the present invention the interconnecting element is a strip with adhesive properties on both sides.

In some exemplary embodiments according to the present invention the second film section was sterilized, or is being sterilized or has been sterilized, together with the first film section.

According to the present invention at least one connection point is a connection point or coupling for a substituate line or a substituate addition site or a coupling.

In some exemplary embodiments according to the present invention the cassette is a blood cassette.

In some exemplary embodiments according to the present invention the method encompasses attaching the first film section and the second film section to the hard body in the same working step or at the same time.

In some exemplary embodiments according to the present invention the method encompasses attaching the first film section and the second film section which were produced integrally with each other.

In some exemplary embodiments according to the present invention the method encompasses providing a perforation line or gaps or slits between the first film section and the second film section of the covering device.

In some exemplary embodiments according to the present invention the method encompasses attaching at least one interconnecting element to the second film section on a side thereof that does not face the connection point.

In some exemplary embodiments according to the present invention the method encompasses both the first film section and the second film section, which are welded, or are being welded, to the hard body of the cassette.

In some exemplary embodiments according to the present invention the method encompasses both the first film section and the second film section, which are first welded, or are first being welded, to the hard body of the cassette and then provided with gaps or a perforation line between them.

In some embodiments according to the present invention, the second film section comprises a section which protrudes from the edge of the cassette.

In further exemplary embodiments according to the present invention, exactly one or only one of the film sections, in particular the one indicated herein as first film section, provided on the cassette or its hard body is limited along its entire circumference by a circumferential sealing bar and/or welding seam or line, whereby the connection point is arranged outside this sealing bar.

In several exemplary embodiments according to the present invention, the connection point is or comprises a liquid addition site.

In some exemplary embodiments according to the present invention, the connection point is not provided under the surface created by the first film section, and therefore not within the circumferential sealing bar.

In further exemplary embodiments according to the present invention, the connection point is provided outside the circumferential sealing bar.

In several exemplary embodiments according to the present invention, the connection point serves as fluid connection, i. e. inlet and/or outlet. It does not comprise or constitute a pump chamber.

In some embodiments according to the present invention, the at least one connection point, which is or will be covered by the covering device, is a connection point, joint or interface for a substituate line.

In several exemplary embodiments according to the present invention, the connection point comprises no check valve.

In several exemplary embodiments according to the present invention, the connection point may represent an opening of the interior of the cassette or of an inner channel of the cassette to the exterior of the cassette (or vice versa).

In some exemplary embodiments according to the present invention, the connection point may not represent the connection between two channels in the interior of the cassette resp. between two channels both covered by the first film section.

In further exemplary embodiments according to the present invention, the connection point may be arranged at the end of an interior channel, i. e. not at a middle section of a channel.

In a further embodiment according to the present invention, the medical fluid-conducting cassette is designed as a blood cassette, for example for dialysis treatment, in particular for hemodialysis, hemofiltration, hemodiafiltration, peritoneal dialysis, acute dialysis, and so on.

Some or all embodiments according to the present invention may comprise one, more or all of the advantages mentioned above and/or hereinafter.

Detaching the contact protection apparatus is advantageously possible without considerable application of force due to the above-mentioned design of the covering device or the second film section.

In some advantageous embodiments, there is a reduced risk of unintentionally contaminating the connection point of the cassette, for example by touching it by hand, as the connection point is safely covered by the second film section.

Since the second film section may extend beyond the rim of the cassette neither the connection point itself nor any point nearby the connection point does have to be touched in order to detach the second film section from the cassette. In fact, using the interconnecting element described herein, the second film section covering the connection point may even be removed automatically, i. e., without any further action having to be taken by the user. In any way, the present invention may contribute to minimizing the risk of unintended contamination during detachment of the device covering the connection point.

The second film section is made from the same material as the first film section needed for covering the hard body and, in some embodiments, for covering or completing the fluid-guiding channels of the cassette. Thus, the second film portion may advantageously undergo the same sterilization processes as the cassette comprising the first film section. Also, since the first film section is cut from a film layer necessarily bigger or differently shaped than the hard body of the cassette, applying the first film section always means that some film material has to be cut off and disposed. According to the present invention, however, parts of this waste film material is being used as the second film section. Hence, in some embodiments according to the present invention providing the second film section does not even require providing additional material. Rather, there are no additional material costs to be born for the second film section.

Detaching the contact protection apparatus from the cassette may in some embodiments be advantageously carried out with just one hand.

Since the second film section is in certain embodiments according to the invention a soft or flexible film material the risk of injury for the user during its operation as well as the risk of damaging other parts of the contact protection apparatus or the cassette, for example by sharp contours, is minimized.

As it is possible to do without the use of adhesives for mounting the second film section to the cassette, no residual adhesive remains at the cassette when the second film section is detached from the cassette and thus no adhesive can penetrate into the interior of the cassette via the connection point.

The present invention is hereafter exemplarily explained by means of the appended drawing, in which identical reference numerals refer to identical or similar components. In the figures it applies that:
- Fig. 1: shows a medical fluid-conducting cassette according to the present invention from one side;
- Fig. 2: shows the medical fluid-conducting cassette of Fig. 1;
- Fig. 3: shows the medical fluid-conducting cassette of Fig. 2;
- Fig. 4: shows a medical fluid-conducting cassette according to another embodiment according to the present invention from one side;
- Fig. 5: shows the medical fluid-conducting cassette in another embodiment according to the present invention; and
- Fig. 6: shows the medical fluid-conducting cassette of Fig. 5 in a transport packaging.

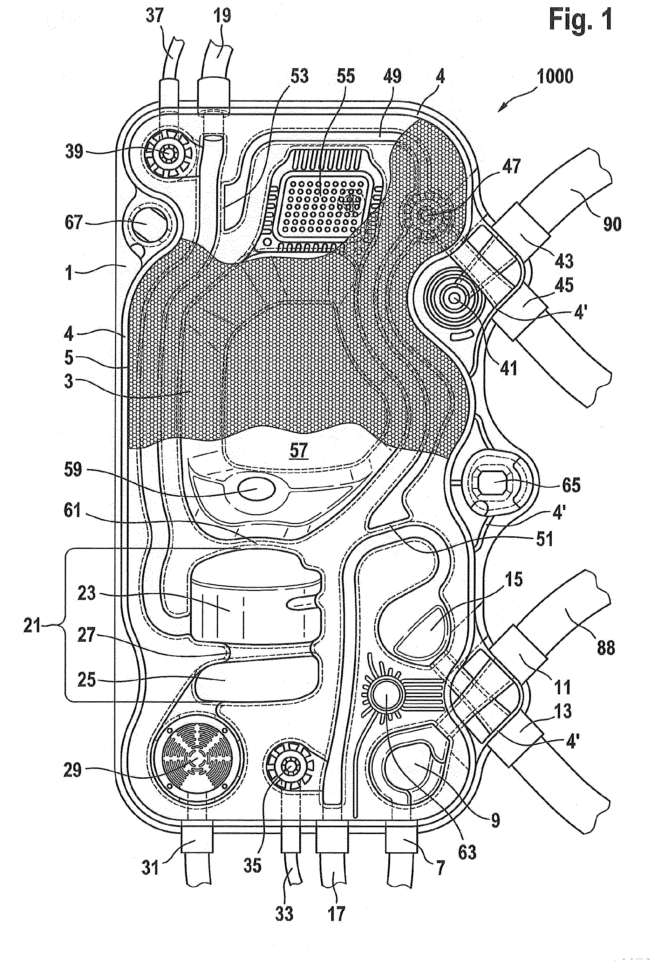

**Fig. 1** shows a possible embodiment of the cassette 1000, here exemplary a medical fluid-conducting cassette 1000 (short: cassette 1000) according to the present invention. The cassette 1000 comprises a hard body 1 comprising channels such as the substituate conduit 49 for guiding a medical fluid such as substituate or blood through the hard part 1.

The hard part 1 is covered by a first film section 3.

The cassette 1000 comprises a connection point or a connection section 41 embodied, in this exemplary embodiment, as a substituate addition site.

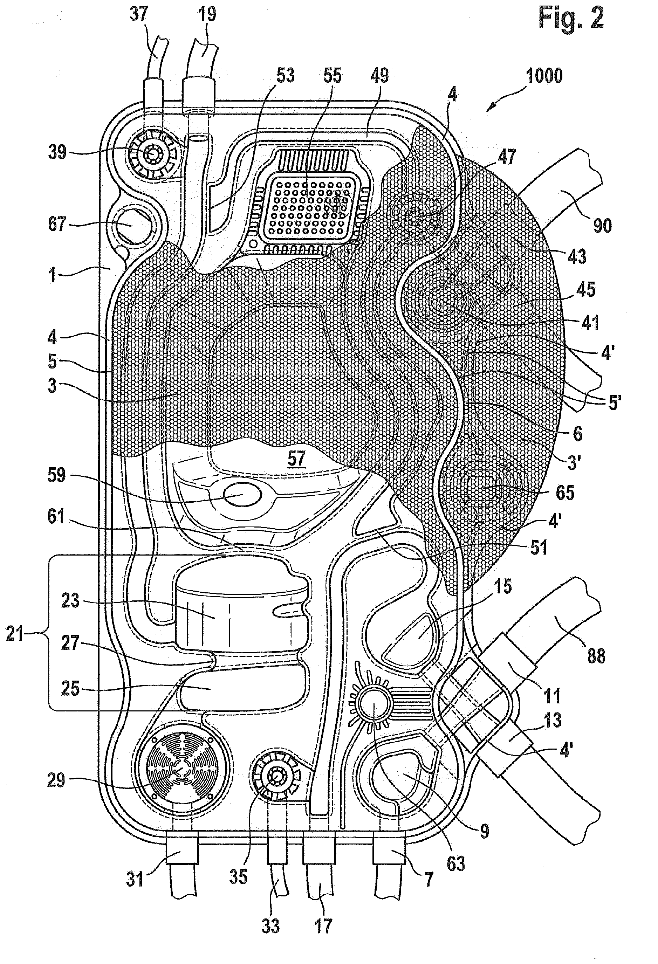

For better understanding only, a second film section that is also comprised by the cassette 1000 according to the present invention is not shown in Fig. 1, but in Fig. 2 to Fig. 4.

As discussed above, the substituate addition site 41 needs to be protected against the environment in a manner such that it remains sterile until use of the cassette 1000.

**Fig. 2** shows the cassette 1000 of Fig. 1. In contrast to Fig. 1, a second film section 3' is illustrated in Fig. 2.

The second film section 3' is made from the same material as the first film section 3. It is arranged so as to cover the connection point, here the substituate addition site 41.

Thus, an unintended touching of the substituate addition site 41 (the connection point) of the cassette 1000 before its use, i.e. before mounting the cassette 1000 to a fluid treatment apparatus is prevented by means of the covering device. Since the second film section 3' covers not only the very spot of the substituate addition site 41, but has been provided with a generous rim or overhang with respect to the hard body 1, unintended touching of the connection point is prevented by the second film section 3' even during removal of it.

As can be seen from Fig. 1 and Fig. 2, the first film section 3 seals the channels of the hard body 1 such as the substituate conduit 49 against an exterior of the cassette 1000 by a peripheral weld by which the first film section 3 is welded to the sealing bar 4. The sealing bar 4 can be a closed structure with no beginning and no end; it may project from the main level of the hard body 1.

Likewise, the second film section 3' can be welded to the hard body 1 as well. The weld can be a closed structure, it may be continuous or intermittent, it may follow both the sealing bar 4 and/or a another bar 4' positioned on opposite sides of the connection point 41 and thus, together with the sealing bar 4, enclosing the connection point 41.

The aforementioned closed structure can be a combination of the sealing bar 4 and the further bar 4'.

Alternatively, or in addition, the second film section 3' can be glued to the hard body 1, preferably in a manner that allows easy removal of the second film section 3' when required.

The first film section 3 and the second film section 3' can be made from a common piece or layer of film. However, between the first film section 3 and the second film section 3' a perforation line 6 can be provided. The perforation may allow tearing the second film section 3' off the first film section 3 or the remainder of the cassette 1000 in order to use the cassette 1000 or the substituate addition site 41.

A perforation may be a combination of a multitude of openings and interposed non-openings (e. g., film material). Likewise, if the second film section 3' is interconnected to the first film section 3 one might also speak of a perforation in accordance with the present invention.

The first film section 3 and the second film section 3' may be interconnected or integral with each other by a perforation which is a sequence of subsequent openings separated from each other by a multitude of film sections in Fig. 2. Alternatively, the first film section 3 and the second film section 3' may be interconnected or integral with each other by a small number of struts or connectors, which are preferably also made from the film material comprised by the first film section 3 and the second film section 3'. For example, two, three or a few more struts or connectors should be enough for keeping the second film section 3' in place. Also, the smaller the number of struts or connectors is the easier it is for the user to remove the second film section 3' when using the cassette 1000.

The cassette 1000 may further comprise one or more of the following elements in any arbitrary combination: A sealing bar 4, a sealing bar 4', a peripheral weld 5, a peripheral weld 5', a perforation line 6, an arterial patient connection 7, an arterial pressure measurement chamber 9, a connector 11 for the exit of blood from cassette 1000, a connector 13 for the entry of blood into cassette 1000; a chamber 15 with arterial post-pump, or pre-filter, pressure measurement site; an arterial filter conduit 17; a venous filter conduit 19; a venous blood chamber 21; an upper space 23 of the venous blood chamber 21; a lower space 25 of the venous blood chamber 21; a cross-sectional restriction 27 of the hard part 1; a clot trap 29; a venous patient connection 31; an arterial addition site 33; a check valve 35 of the arterial addition site 33; a venous addition site 37; a check valve 39 of the venous addition site 37; a connector 43 for exit of substituate from the cassette 1000; a connector 45 for entry of substituate into the cassette 1000; a check valve 47 for addition of substituate; a pre-dilution addition valve 51 (phantom valve); a post-dilution addition valve 53 (phantom valve); a single-needle sterile membrane 55; a single-needle chamber 57; a blood surge redirection element 59; a single-needle blood valve 61 (phantom valve); an evacuation site 63 for vacuum coupling; a primary alignment center 65; a secondary alignment site 67; a pump tube segment 88 of blood pump; and a pump tube segment 90 of substituate pump.

Either one of sealing bar 4 and sealing bar 4' may protrude from the hard body 1 or from the level of the first film section 3.

In Fig. 2 the sealing bar 4 is shown not to be covered by a film at all. The invention is, however, not limited to this. Rather, the film layer of which the first film section 3 and the second film section 3' are made may cover the sealing 4 as well. Any perforation, gaps or the like may, for example, be arranged on top or next to the sealing bar 4.

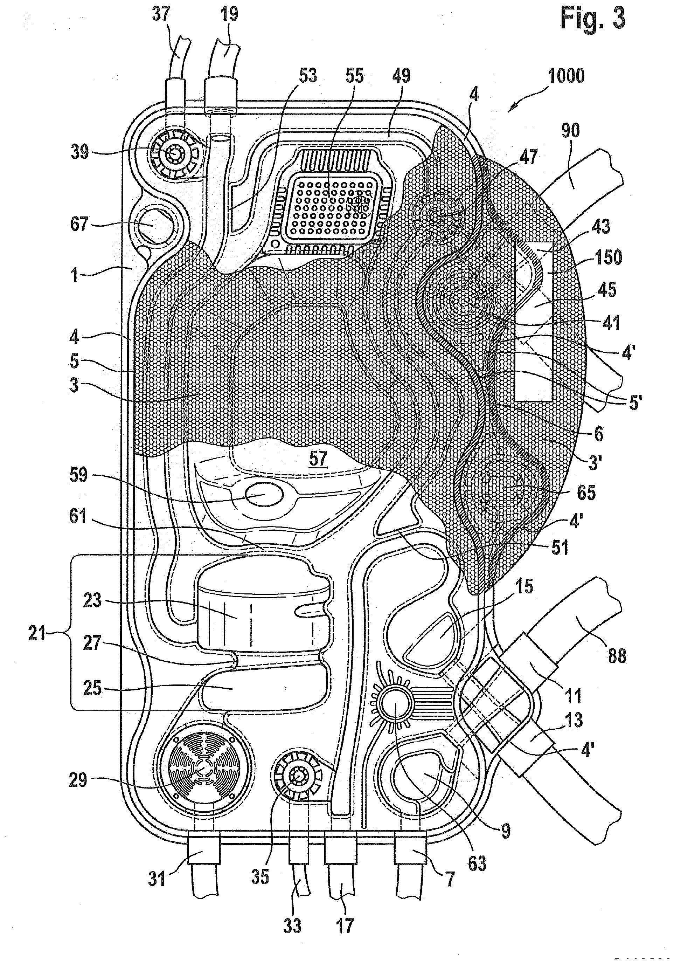

**Fig. 3** shows the medical fluid-conducting cassette of Fig. 2. What is highlighted in Fig. 3 is the path along which the second film section 3' can be welded to the hard body 1 of the cassette 1000.

As can be seen in Fig. 3, the welding path can be or may circumscribe a closed structure, surrounding the connection point 41 in a closed manner or loop. It may, as in Fig. 3, have the shape of the number "8".

Also, in Fig. 3 as may be the case in any other embodiment according to the present invention an interconnecting element 150 is connected to the second film section 3'.

The interconnecting element 150 can be an adhesive, a sticky tape, a Velcro fastener, a tape that is adhesive on both sides or double-sided adhesive tape, or the like.

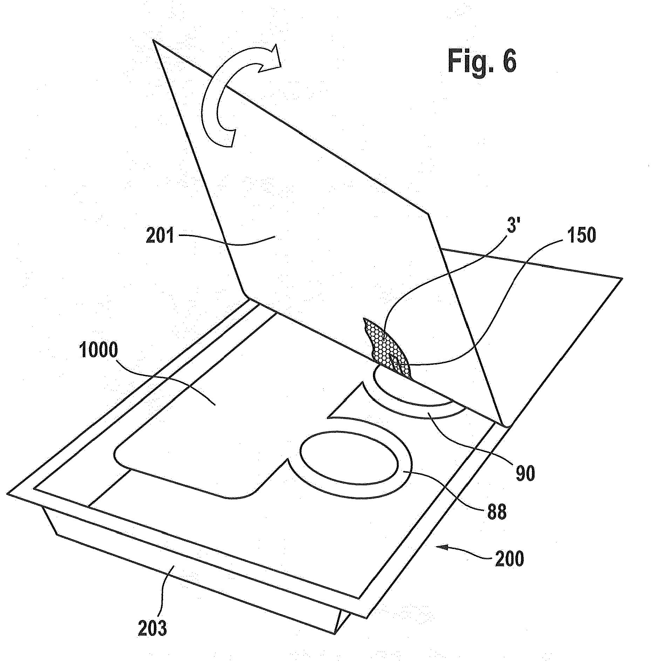

The interconnecting element 150 serves to keep the second film section 3' securely to a lid 201 of a transport packaging 200 only shown in Fig. 6 and is, hence, discussed only with respect to Fig. 6.

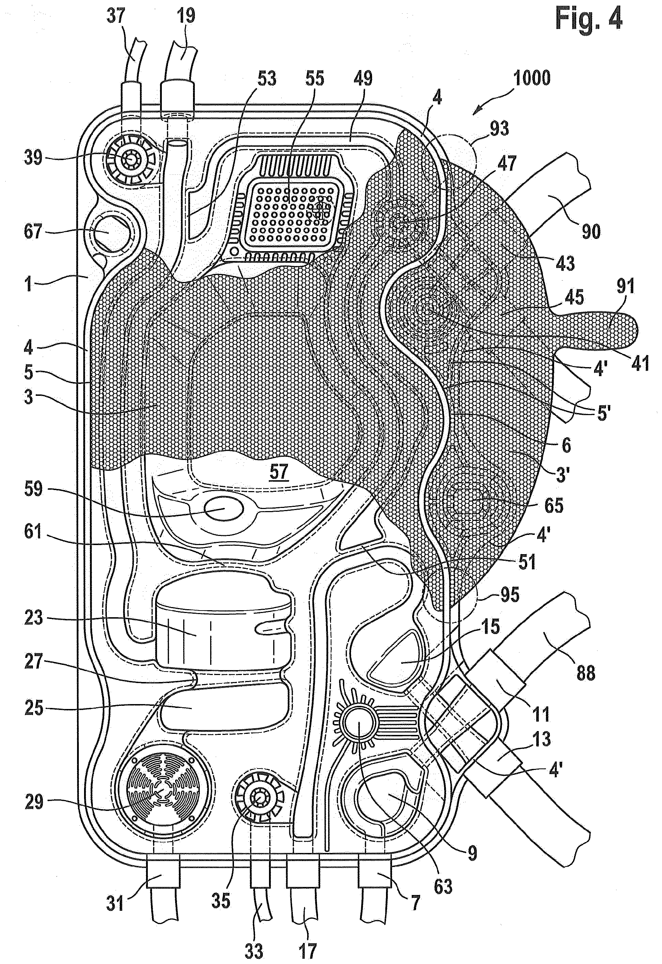

**Fig. 4** shows a medical fluid-conducting cassette 1000 according to another embodiment according to the present invention.

The second film section 3' comprises a flap or removal flap 91 which extends from a rim of the second film section 3' and may facilitate getting a grip on the second film section 3' in order to tear it off the cassette 1000.

Also, two end sections or edge sections 93 and 95 are emphasized by circles in Fig. 4. At sections 93 and 95 the sealing bar 4' joins with the sealing bar 4. In the example of Fig. 4, this junction is where the second film section 3' ends. In other embodiments according to the present invention, there is no such junction, but the second film section 3' ends nevertheless in some end sections.

At at least one of its end or edge sections 93, 95, the second film section 3' may be connected to the first film section 3 in a manner which differs from how the two film sections 3, 3' are interlinked with each other. For example, in either of sections 93, 95, the two film sections 3, 3' may be integral without perforation between them. That way, the second film section 3' may be firmly connected to the first film section 3. A perforation between them, e. g., between the two edge sections 93, 95 may not be needed in those embodiments. In particular, the two film sections 3, 3' may be cut into two separate pieces between the two edge sections 93, 95 that are solely responsible for keeping the second film section 3' to the first film 3 or for connecting them with each other.

In some embodiments according to the present invention, the edge sections 93, 95 are chosen such that they do not cover a rib of the hard body 1 and/or are not supported by a rib or other hard body structure from underneath.

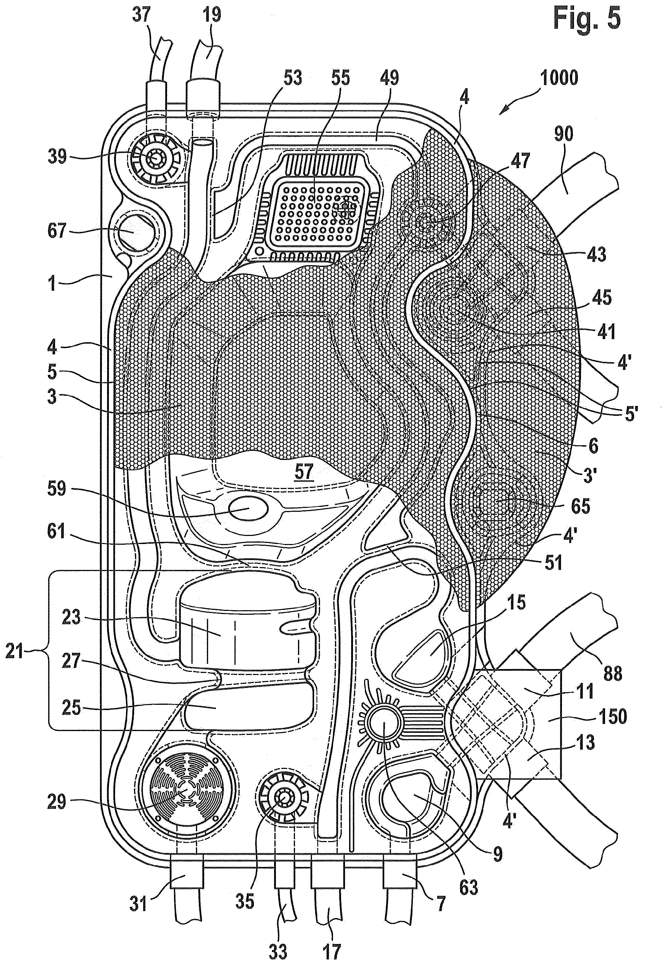

**Fig. 5** shows a medical fluid-conducting cassette 1000 in yet another exemplary embodiment of the present invention.

Another interconnecting element 150 is connected to the second film section 3'. It may be provided as a substitute or an alternative to the one shown in Fig. 3.

Again, the interconnecting element 150 can be an adhesive, a sticky tape, a Velcro fastener, a tape that is adhesive on both sides or double-sided adhesive tape, or the like.

The interconnecting element 150 serves to keep the second film section 3' securely to a lid 201 of a transport packaging 200 only shown in Fig. 6 and is, hence, again further discussed with respect to Fig. 6.

**Fig. 6** shows a transport packaging 200. The transport packaging 200 comprises a lid 201 covering or sealing a reception 203 of the transport packaging 200. The reception 203 holds or houses a cassette 1000 like that of Fig. 3. The cassette 1000 comprises the interconnecting element 150 mentioned above.

As can be derived from the illustration of Fig. 6, the interconnecting element 150 has obviously been attached to the lid 201 during the packing of the cassette 1000 into the transport packaging 200. Also, as is indicated by the arrow indicating that the lid 201 is being opened in Fig. 6, the interconnecting element 150 does not only stick to the lid 201 but also lifts or takes the second film section 3' with it which in turn is attached to the interconnecting element 150. That way the second film section 3' is removed from the cassette 1000 merely by opening the lid 201 of the transport packaging 200 upon opening the latter.

### Reference numerals

- 1000: cassette
- 1: hard part or hard body
- 3: first film section
- 3': second film section
- 4: sealing bar
- 4': sealing bar
- 5: peripheral weld
- 5': peripheral weld
- 6: perforation line
- 7: arterial patient connection
- 9: arterial pressure measurement chamber
- 11: connector for the exit of blood from cassette 1000, or: blood outlet connector
- 13: connector for the entry of blood into cassette 1000, or: blood inlet connector
- 15: chamber with arterial post-pump, or pre-filter, pressure measurement site
- 17: arterial filter conduit
- 19: venous filter conduit
- 21: venous blood chamber
- 23: upper space of the venous blood chamber 21
- 25: lower space of the venous blood chamber 21
- 27: cross-sectional restriction of the hard body 1
- 29: clot trap
- 31: venous patient connection
- 33: arterial addition site
- 35: check valve of the arterial addition site 33
- 37: venous addition site
- 39: check valve of the venous addition site 37
- 41: substituate addition site, connection point
- 43: connector for exit of substituate from the cassette 1000
- 45: connector for entry of substituate into the cassette 1000
- 47: check valve for addition of substituate
- 49: substituate conduit; channel
- 51: pre-dilution addition valve (phantom valve)
- 53: post-dilution addition valve (phantom valve)
- 55: single-needle sterile membrane
- 57: single-needle chamber
- 59: blood surge redirection element
- 61: single-needle blood valve (phantom valve)
- 63: evacuation site for vacuum coupling
- 65: primary alignment center
- 67: secondary alignment site
- 88: pump tube segment of blood pump
- 90: pump tube segment of substituate pump

- 91: flap
- 93: end sections or edge section
- 95: end sections or edge section

- 150: interconnecting element
- 200: transport packaging
- 201: lid
- 203: reception

## Claims

1. A medical fluid-conducting cassette (1000) for a medical fluid treatment, comprising:
- a hard body (1) comprising channels (49) for guiding fluids through the cassette (1000);
- a first film section (3) arranged to cover parts of the hard body (1) and arranged to cover at least one of the channels (49) against an exterior of the blood cassette (1000);
- at least one connection point (41) configured to connect at least one of the channels (49) with an exterior of the blood cassette (1000) in a fluid communication by connecting the connection point (41) with a tubing or a substituate line or a substituate site or a coupling;
- at least one covering device attached to the hard body (1) and arranged so as to cover the connection point (41),
wherein the covering device is or comprises a second film section (3') made from the same material as the first film section (3),
**characterised in that**
the covering device is attached to the hard body (1) to be releasable or removable from the hard body (1) without any tools.

2. The medical fluid-conducting cassette (1000) according to claim 1, wherein the first film section (3) and the second film section (3') are integrally formed or produced.

3. The medical fluid-conducting cassette (1000) according to one of the preceding claims, wherein the first film section (3) and the second film section (3') are linked one to the other by at least one perforation section (6) and/or at least two connectors integral with both the first film section (3) and the second film section (3'), the connectors preferably being spaced from each other.

4. The medical fluid-conducting cassette (1000) according to one of the preceding claims, wherein the second film section (3') is welded to the hard body (1).

5. The medical fluid-conducting cassette (1000) according to one of the preceding claims, wherein the first film section (3) and the second film section (3') have been welded to the hard body (1), wherein the welding methods or parameters used for the two film sections (3, 3') differ for each of the sections (3, 3').

6. The medical fluid-conducting cassette (1000) according to one of the preceding claims, wherein the second film section (3') comprises an interconnecting element (150) configured for interconnecting the second film section (3') to a transport packaging (200) of the cassette (1000).

7. The medical fluid-conducting cassette (1000) according to claim 6, packed into the transport packaging (200), wherein the second film section (3') is interconnected to the transport packaging (200) of the cassette (1000) by the interconnecting element (150).

8. The medical fluid-conducting cassette (1000) according to one of claims 6 to 7, wherein the interconnecting element (150) is an adhesive strip, material or layer.

9. The medical fluid-conducting cassette (1000) according to one of claims 6 to 8, wherein the interconnecting element (150) is a strip with adhesive properties on both sides.

10. The medical fluid-conducting cassette (1000) according to one of the preceding claims, wherein at least one connection point (41) is a connection point for a substituate line or a substituate addition site.

11. The medical fluid-conducting cassette (1000) according to one of the preceding claims, embodied as a blood cassette.

12. A method for covering a hard body (1) of a medical fluid-conducting cassette (1000) for a medical fluid treatment by a film material, the method comprising the steps:
- providing a cassette, the cassette (1000) comprising:
- a hard body (1) comprising channels (49) for guiding fluids through the cassette (1000);
- at least one connection point (41) configured to connect at least one of the channels (49) with an exterior of the blood cassette (1000) in a fluid communication by connecting the connection point (41) with a tubing or a substituate line or a substituate site or a coupling;
- attaching a first film section (3) to the hard body (1) so as to cover parts of the hard body (1) and so as to cover at least one of the channels (49) against an exterior of the blood cassette (1000);
- attaching a second film section (3') to the hard body (1) so as to cover the connection point (41) in a releasable or removable manner, the second film section (3') comprising the same material as the first film section (3) or consisting thereof,
**characterised in that**
the covering device is being attached to the hard body (1) to be releasable or removable from the hard body (1) without any tools.

13. The method according to claim 12, wherein the first film section (3) and the second film section (3') are being attached to the hard body (1) in the same working step and/or wherein the first film section (3) and the second film section (3') were produced integrally with each other.

14. The method according to claim 12 or claim 13, comprising the step:
- providing or generating a perforation line (6) or gaps or slits between the first film section (3) and the second film section (3') of the covering device.

15. The method according to claim 12, 13 or 14, comprising the step:
- attaching at least one interconnecting element (150) to the second film section (3') on a side thereof that does not face the connection point (41).

## Patentansprüche

1. Medizinische fluidführende Kassette (1000) für eine medizinische Fluidbehandlung, umfassend:
- ein hartes Gehäuse (1) mit Kanälen (49) zum Leiten von Fluiden durch die Kassette (1000);
- einen ersten Filmabschnitt (3), der so angeordnet ist, dass er Teile des harten Gehäuses (1) abdeckt, und so angeordnet ist, dass er mindestens einen der Kanäle (49) gegen eine Außenseite der Blutkassette (1000) abdeckt;
- mindestens einen Verbindungspunkt (41), der so konfiguriert ist, dass er mindestens einen der Kanäle (49) mit einem Äußeren der Blutkassette (1000) in einer Fluidverbindung verbindet, indem er den Verbindungspunkt (41) mit einem Schlauch oder einer Substituatleitung oder einer Substituatzugabestelle oder einer Kupplung verbindet;
- mindestens eine Deckvorrichtung, die an dem harten Gehäuse (1) befestigt und so angeordnet ist, dass sie den Verbindungspunkt (41) abdeckt,
wobei die Deckvorrichtung ein zweiter Filmabschnitt (3') ist oder einen zweiten Filmabschnitt (3') aufweist, der aus dem gleichen Material wie der erste Filmabschnitt (3) hergestellt ist,
**dadurch gekennzeichnet, dass**
die Deckvorrichtung an dem harten Gehäuse (1) so befestigt ist, dass sie ohne Werkzeug von dem harten Gehäuse (1) gelöst oder entfernt werden kann.

2. Die medizinische fluidführende Kassette (1000) nach Anspruch 1, wobei der erste Filmabschnitt (3) und der zweite Filmabschnitt (3') einstückig geformt oder hergestellt sind.

3. Die medizinische fluidführende Kassette (1000) nach einem der vorhergehenden Ansprüche, wobei der erste Filmabschnitt (3) und der zweite Filmabschnitt (3') durch mindestens einen Perforationsabschnitt (6) und/oder mindestens zwei sowohl mit dem ersten Filmabschnitt (3) als auch mit dem zweiten Filmabschnitt (3') einstückig ausgebildete Verbinder miteinander verbunden sind, wobei die Verbinder vorzugsweise voneinander beabstandet sind.

4. Die medizinische fluidführende Kassette (1000) nach einem der vorhergehenden Ansprüche, wobei der zweite Filmabschnitt (3') mit dem harten Gehäuse (1) verschweißt ist.

5. Die medizinische fluidführende Kassette (1000) nach einem der vorhergehenden Ansprüche, wobei der erste Filmabschnitt (3) und der zweite Filmabschnitt (3') an den harten Körper (1) geschweißt sind, wobei die für die beiden Filmabschnitte (3, 3') verwendeten Schweißverfahren oder Schweißparameter für jeden der Abschnitte (3, 3') unterschiedlich sind.

6. Die medizinische fluidführende Kassette (1000) nach einem der vorhergehenden Ansprüche, wobei der zweite Filmabschnitt (3') ein Verbindungselement (150) aufweist, das zum Verbinden des zweiten Filmabschnitts (3') mit einer Transportverpackung (200) der Kassette (1000) ausgebildet ist.

7. Die medizinische fluidführende Kassette (1000) nach Anspruch 6, verpackt in der Transportverpackung (200), wobei der zweite Filmabschnitt (3') durch das Verbindungselement (150) mit der Transportverpackung (200) der Kassette (1000) verbunden ist.

8. Die medizinische fluidführende Kassette (1000) nach einem der Ansprüche 6 bis 7, wobei das Verbindungselement (150) ein Klebestreifen, ein Klebematerial oder eine Klebstoffschicht ist.

9. Die medizinische fluidführende Kassette (1000) nach einem der Ansprüche 6 bis 8, wobei das Verbindungselement (150) ein Streifen mit Klebeeigenschaften auf beiden Seiten ist.

10. Die medizinische fluidführende Kassette (1000) nach einem der vorhergehenden Ansprüche, wobei mindestens ein Verbindungspunkt (41) ein Verbindungspunkt für eine Substituatleitung oder eine Substituatzugabestelle ist.

11. Die medizinische fluidführende Kassette (1000) nach einem der vorhergehenden Ansprüche, ausgeführt als Blutkassette.

12. Verfahren zum Abdecken eines harten Gehäuses (1) einer medizinischen fluidführenden Kassette (1000) für eine medizinische Fluidbehandlung durch ein Filmmaterial, wobei das Verfahren die Schritte umfasst:
- Bereitstellen einer Kassette, wobei die Kassette (1000) umfasst:
- ein hartes Gehäuse (1) mit Kanälen (49) zum Führen von Fluiden durch die Kassette (1000);
- mindestens einen Verbindungspunkt (41), der so konfiguriert ist, dass er mindestens einen der Kanäle (49) mit einem Äußeren der Blutkassette (1000) in einer Fluidverbindung verbindet, indem er den Verbindungspunkt (41) mit einem Schlauch oder einer Substituatleitung oder einer Substituatzugabestelle oder einer Kupplung verbindet;
- Anbringen eines ersten Filmabschnitts (3) an dem harten Gehäuse (1), um Teile des harten Gehäuses (1) abzudecken und um mindestens einen der Kanäle (49) gegen eine Außenseite der Blutkassette (1000) abzudecken;
- Anbringen eines zweiten Filmabschnitts (3') an dem harten Gehäuse (1), um den Verbindungspunkt (41) lösbar oder entfernbar abzudecken, wobei der zweite Filmabschnitt (3') dasselbe Material wie der erste Filmabschnitt (3) aufweist oder daraus besteht,
**dadurch gekennzeichnet, dass**
die Deckvorrichtung an dem harten Gehäuse (1) so angebracht wird, dass sie ohne Werkzeug von dem harten Gehäuse (1) gelöst oder entfernt werden kann.

13. Das Verfahren nach Anspruch 12, wobei der erste Filmabschnitt (3) und der zweite Filmabschnitt (3') im gleichen Arbeitsschritt am harten Gehäuse (1) befestigt werden und/oder wobei der erste Filmabschnitt (3) und der zweite Filmabschnitt (3') einstückig hergestellt wurden.

14. Das Verfahren nach Anspruch 12 oder Anspruch 13, umfassend den Schritt:
- Bereitstellen oder Erzeugen einer Perforationslinie (6) oder von Lücken oder Schlitzen zwischen dem ersten Filmabschnitt (3) und dem zweiten Filmabschnitt (3') der Deckvorrichtung.

15. Das Verfahren nach Anspruch 12, 13 oder 14, umfassend den Schritt:
- Anbringen von mindestens einem Verbindungselement (150) an dem zweiten Filmabschnitt (3') auf einer Seite desselben, die nicht dem Verbindungspunkt (41) zugewandt ist.

## Revendications

1. Une cassette conductrice de fluide médical (1000) pour un traitement de fluide médical, comprenant:
- un corps rigide (1) comprenant des canaux (49) pour guider des fluides au travers de la cassette (1000);
- une première section de film (3) agencée pour recouvrir des parties du corps rigide (1) et agencée pour recouvrir au moins un des canaux (49) contre un extérieur de la cassette (1000);
- au moins un point de connexion (41) configuré pour relier au moins un des canaux (49) avec un extérieur de la cassette (1000) en communication fluidique en reliant le point de connexion (41) à un tuyau, ou à un conduit de substitut, ou à un site de substitut, ou à un raccord;
- au moins un dispositif de recouvrement attaché au corps rigide (1) et agencé de façon à recouvrir le point de connexion (41);
où le dispositif de recouvrement est ou comprend une seconde section de film (3') constituée du même matériau que la première section de film (3),
**caractérisée en ce que**
le dispositif de recouvrement est attaché au corps rigide (1) de façon à être séparable ou détachable du corps rigide (1) sans aucun outil.

2. La cassette conductrice de fluide médical (1000) selon la première revendication, où la première section de film (3) ainsi que la seconde section de film (3') sont formées ou produites intégralement.

3. La cassette conductrice de fluide médical (1000) selon l'une quelconque des revendications précédentes, où la première section de film (3) ainsi que la seconde section de film (3') sont reliées l'une à l'autre par au moins une section de perforation (6) et/ou au moins deux connecteurs intégrés à la fois à la première section de film (3) ainsi qu'à la seconde section de film (3'), les connecteurs étant de préférence espacés l'un de l'autre.

4. La cassette conductrice de fluide médical (1000) selon l'une quelconque des revendications précédentes, où la seconde section de film (3') est soudée au corps rigide (1).

5. La cassette conductrice de fluide médical (1000) selon l'une quelconque des revendications précédentes, où la première section de film (3) et la seconde section de film (3') ont été soudées au corps rigide (1), où les procédés ou les paramètres de soudage utilisés pour les deux sections de film (3, 3') différent pour chacune des sections (3, 3').

6. La cassette conductrice de fluide médical (1000) selon l'une quelconque des revendications précédentes, où la seconde section de film (3') comprend un élément d'interconnexion (150) configuré pour interconnecter la seconde section de film (3') à un emballage de transport (200) de la cassette (1000).

7. La cassette conductrice de fluide médical (1000) selon la revendication 6, emballée dans l'emballage de transport (200), où la seconde section de film (3') est interconnectée à l'emballage de transport (200) de la cassette (1000) via l'élément d'interconnexion (150).

8. La cassette conductrice de fluide médical (1000) selon l'une quelconque des revendications 6 à 7, où l'élément d'interconnexion (150) est une bande, un matériau ou une couche adhésif/ve.

9. La cassette conductrice de fluide médical (1000) selon l'une quelconque des revendications 6 à 8, où l'élément d'interconnexion (150) est une bande avec des propriétés adhésives des deux côtés.

10. La cassette conductrice de fluide médical (1000) selon l'une quelconque des revendications précédentes, où au moins un point de connexion (41) est un point de connexion pour un conduit de substitut ou pour un site d'ajout de substitut.

11. La cassette conductrice de fluide médical (1000) selon l'une quelconque des revendications précédentes, conçue sous la forme d'une cassette à sang.

12. Un procédé pour recouvrir un corps rigide (1) d'une cassette conductrice de fluide médical (1000) pour un traitement de fluide médical par un matériau pelliculaire, le procédé comprenant les étapes suivantes:
- la fourniture d'une cassette, la cassette (1000) comprenant:
- un corps rigide (1) comprenant des canaux (49) pour guider des fluides au travers de la cassette (1000);
- au moins un point de connexion (41) configuré pour relier au moins un des canaux (49) avec un extérieur de la cassette (1000) en communication fluidique en reliant le point de connexion (41) à un tuyau, ou à un conduit de substitut, ou à un site de substitut, ou à un raccord;
- la fixation d'une première section de film (3) au corps rigide (1) de façon à recouvrir des parties du corps rigide (1) et à recouvrir au moins un des canaux (49) contre un extérieur de la cassette (1000);
- la fixation d'une seconde section de film (3') au corps rigide (1) de façon à recouvrir le point de connexion (41) de manière séparable ou détachable, la seconde section de film (3') comprenant le même matériau que la première section de film (3) ou consistant en celle-ci,
**caractérisé en ce que**
le dispositif de recouvrement est fixé au corps rigide (1) de façon à être séparable ou détachable du corps rigide (1) sans aucun outil.

13. Le procédé selon la revendication 12, où la première section de film (3) et la seconde section de film (3') sont attachées au corps rigide (1) dans la même étape de travail et/ou où la première section de film (3) et la seconde section de film (3') ont été produites intégralement l'une avec l'autre.

14. Le procédé selon la revendication 12 ou 13, comprenant l'étape suivante:
- la fourniture ou la génération d'un conduit de perforation (6), ou d'espaces, ou de fentes entre la première section de film (3) et la seconde section de film (3') du dispositif de recouvrement.

15. Le procédé selon la revendication 12, 13 ou 14, comprenant l'étape suivante:
- la fixation d'au moins un élément d'interconnexion (150) à la seconde section de film (3') sur un côté de celle-ci ne faisant pas face au point de connexion (41).
